# EUROPEAN PATENT SPECIFICATION

(11) **EP 1 491 168 B1**
(45) Date of publication and mention of the grant of the patent: **04.11.2009**
(21) Application number: 04253833.0
(22) Date of filing: 24.06.2004
(51) Int. Cl.: A61F 5/00, A61F 2/00, A61B 17/12

(54) **Implantable band with attachment mechanism having dissimilar material properties**
Implantierbares Band mit einer Verbindungsvorrichtung bestehend aus Materialien mit unterschiedlichen Materialeigenschaften
Bande implantable avec mécanisme de fixation en matériaux avec propriétés différentes

(30) Priority: 27.06.2003 US 483353 P; 30.09.2003 US 507612 P
(43) Date of publication of application: 29.12.2004
(73) Proprietor: ETHICON ENDO-SURGERY, INC., Cincinnati, Ohio 45242 (US)
(72) Inventor: Byrum, Randal, King Mills, OH 45034 (US); Wiley, Jeffrey P., Milford, OH 45150 (US); Conlon, Sean P., Loveland, OH 45140 (US); Fender, Bill, Springboro, OH 45140 (US); Tsonton, Mark, Loveland, OH 45140 (US); Ortiz, Mark, Milford, OH 45150 (US); Dunki-Jacobs, Adam, Columbus, OH 43201 (US)
(74) Representative: Tunstall, Christopher Stephen

(56) References cited:
- FR-A- 2 434 089
- US-A- 3 576 054
- US-A- 3 860 997
- US-A- 4 854 014
- US-A- 5 363 536

## Description

### Technical Field

This present invention relates generally to a surgically implantable band for encircling an anatomical passageway, and is particularly directed to an adjustable gastric band for encircling the stomach for the control of obesity. The invention will be specifically disclosed in connection with an improved attachment mechanism for an adjustable gastric band.

### Background Of The Invention

Since the early 1980s, adjustable gastric bands have provided an effective alternative to gastric bypass and other irreversible surgical weight loss treatments for the morbidly obese. The gastric band is wrapped around an upper portion of the patient's stomach, forming a stoma that is less than the normal interior diameter of the stomach that restricts food passing from an upper portion to a lower digestive portion of the stomach. When the stoma is of the appropriate size, food held in the upper portion of the stomach provides a feeling of fullness that discourages overeating.

In addition to a latched position to set the diameter of the gastric band, adjustability of gastric bands is generally achieved with an inwardly directed inflatable balloon, similar to a blood pressure cuff, into which fluid, such as saline, is injected through a fluid injection port to achieve a desired diameter. The balloon is typically deflated or only partially inflated when first placed in the body to allow for body adjustments and healing around the new band site. Since adjustable gastric bands may remain in the patient for long periods of time, the fluid injection port is typically installed subcutaneously to avoid infection, for instance in front of the sternum. Following the initial implantation, the surgeon may adjust the band by loosing or tightening depending on the patients' needs. Adjusting the amount of fluid in the adjustable gastric band is achieved by inserting a Huber tip needle through the skin into a silicone septum of the injection port. Once the needle is removed, the septum seals against the hole by virtue of compressive load generated by the septum. A flexible conduit communicates between the injection port and the adjustable gastric band.

An attachment mechanism for the adjustable gastric band has to provide an initial sizing of the stoma of the stomach. One generally known attachment is to suture ends of the adjustable gastric band. Another generally known attachment includes one end of the gastric band terminating in a flexible conduit that has a flared portion that is drawn through an opening in a second end of the gastric band and then sutured to the encircling band portion - securing the band to the stomach. After the sutures are in place, the injection port is anchored at a convenient location.

United States patent no. 5,938,669 discloses an adjustable gastric banding device having a band connected by a tube to a control box and a balancing reservoir implanted under a patient's skin, the box containing an electric pump and an electronic control unit capable of communicating by radio with a monitor carried by the patient and with a controller intended for the doctor. The controller can operate the pump by remote control to transfer determined volumes of liquid in a closed circuit from the band to the reservoir or vice versa, to adjust the diameter of a passage in the stomach. United States patent no. 5,226,429 discloses a gastric band adapted for laparoscopic placement around the stomach and a method for deploying the band. Cameras are used for observing the placement of the band. The band is secured against slippage after being placed at the desired position.
European patent application publication no. 1 319 371 discloses a strip to be implanted surgically, to form a restrictive ring around the stomach, has a closure near the transit between the feed tube and the loop section. The free end of the loop section has threading length for the closure, with lateral beads at the end, and a plug with a locking hook to fit into the eyelet of the closure.
International patent application publication no. WO 2004/108025 represents a state of the art document according to Art. 54(3) EPC and discloses a surgical ring comprising a flexible band and a closure means which has male and female elements for connecting two ends of the band. The female element has a structural discontinuity at which the deformability of the female element is greater than the rest of the female element.
European patent application publication no. 1 396 242 represents a state of the art document according to Art. 54(3) EPC and discloses a surgical ring comprising a flexible band and a closure means for forming a closed loop. The band and the closure means preferably form a single piece of the same material.

While these known approaches are effective in securing the gastric band, further improvements are desired that simplify the clinical implantation procedure, that provide long-term reliability, and that facilitate readjustment or removal.

While sutures have been relied on as the most positive connection in the past, it is desirable to have a secure attachment that does not require sutures, yet does not require a large force to create the secure attachment. Otherwise, it may be difficult to adequately grip and perform the attachment with laparoscopic instruments. Consequently, a significant need exists for an adjustable gastric band having an improved attachment mechanism.

### Summary of The Invention

The present invention addresses these and other problems in the prior art, by providing an adjustable gastric band device that is engaged with less force, thereby facilitating implementation with laparoscopic instruments, yet the attachment remains secure over long term use.

A general object of this invention is to provide an adjustable gastric band which comprises material having at least one first material property and having an attachment mechanism which comprises material that has at least one second material property corresponding to but different from the first material property. The attachment mechanism may include plastically or elastically deformable material.

Another object of this invention is to provide a readily reversible adjustable gastric band which can be fastened and unfastened without reducing the holding strength of the attachment mechanism.

A still further object of this invention is to provide an attachment mechanism requiring a light force to latch and a high force to unlatch the ends.

### Brief Description Of The Figures

The accompanying drawings, which are incorporated in and constitute a part of this specification, illustrate embodiments of the invention, and, together with the general description of the invention given above, and the detailed description of the embodiments given below, serve to explain the principles of the present invention.

FIG. 1 is a diagrammatic drawing showing an adjustable gastric band wrapped around an upper part of a stomach.

FIG. 2 is a cross sectional view of the adjustable gastric band of FIG 1 taken along line 2-2.

FIG. 3 is a top plan view of an adjustable gastric band having an attachment mechanism comprising dissimilar, plastically deformable material.

FIG. 4 is an enlarged fragmentary view of the end portions of the band shown in Fig. 3.

FIG. 5 is cross sectional view taken along line 5-5 of FIG. 4.

FIG. 6 is cross sectional view taken along line 6-6 of FIG. 4.

FIG. 7 is cross sectional view taken along line 7-7 of FIG. 6.

FIG. 8 is a top plan view in partial cross section of another embodiment of an adjustable gastric band having an attachment mechanism comprising dissimilar, elastically deformable material.

FIG. 9 is an enlarged fragmentary view of the attachment mechanism of FIG. 8.

FIG. 10 is an enlarged fragmentary cross sectional view similar to FIG. 9 of another embodiment of an attachment mechanism comprising dissimilar, elastically deformable material.

FIG. 11 is an enlarged fragmentary top plan view in cross section of another embodiment of an attachment mechanism comprising dissimilar, elastically deformable material.

FIG. 11A is an end view of the receiver shown in FIG. 11.

FIG. 12 is an enlarged fragmentary top plan view in cross section of the embodiment depicted in FIG. 11, showing the attachment mechanism connected together.

FIG. 13 is an enlarged fragmentary perspective view of another embodiment of an attachment mechanism comprising dissimilar, elastically deformable material.

FIG. 14 is an enlarged fragmentary perspective view of the embodiment shown in FIG. 13, showing the attachment mechanism connected together.

FIG. 15 is an enlarged cross section taken along line 15-15 of FIG. 14.

FIG. 16 is an enlarged exploded perspective view of another embodiment of an attachment mechanism comprising dissimilar material including an elastically deformable component.

FIG. 17 is an enlarged perspective view in cross section of the housing shown in FIG. 16.

FIGS. 18-20 are views similar to FIG. 17, with the insert disposed in the housing, showing various positions of the insert during operation.

FIGS. 21-23 are top views of the attachment mechanism shown in FIGS. 16-20.

FIG. 24 is an enlarged fragmentary perspective view of another embodiment of an attachment mechanism comprising dissimilar, elastically deformable material.

FIG. 25 is an enlarged fragmentary perspective view of the embodiment shown in FIG. 24, showing the attachment mechanism connected together.

FIG. 26 is enlarged fragmentary perspective view of the embodiment shown in FIG. 24, showing the attachment mechanism in an intermediate stage of disconnecting the ends of the adjustable gastric band.

Reference will now be made in detail to the present preferred embodiment of the invention, an example of which is illustrated in the accompanying drawings.

### Detailed Description of Embodiments of the Invention

In the following description, like reference characters designate like or corresponding parts throughout the several views. Also, in the following description, it is to be understood that terms such as front, back, inside, outside, and the like are words of convenience and are not to be construed as limiting terms. Terminology used in this patent is not meant to be limiting insofar as devices described herein, or portions thereof, may be attached or utilized in other orientations. Referring in more detail to the drawings, the invention will now be described.

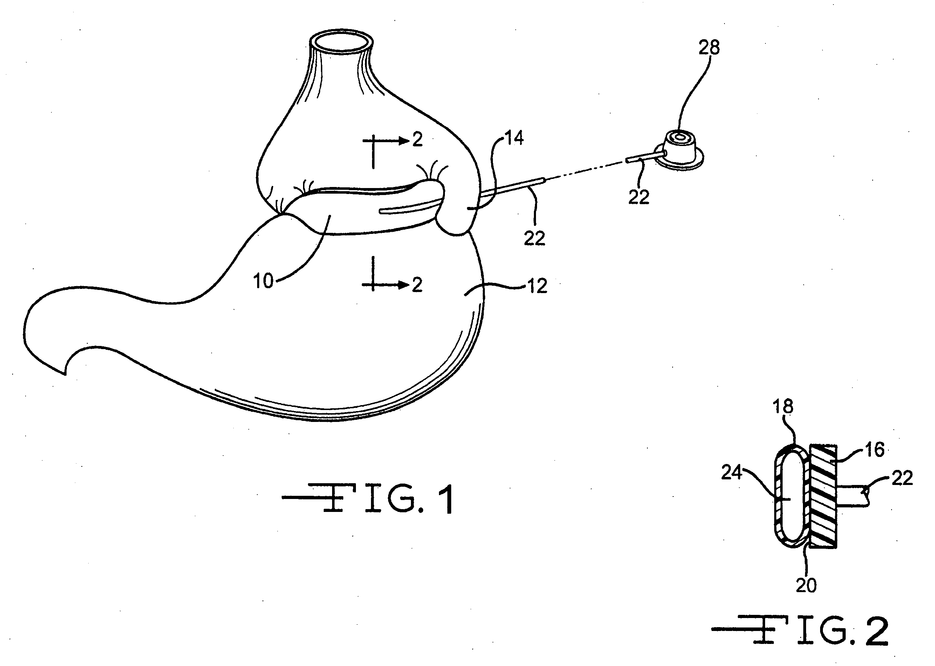

Referring to Fig. 1, an adjustable gastric band 10 is shown wrapped around an upper portion of a stomach 12, kept in place by attaching the two ends together and extending a portion 14 of the stomach 12 over the adjustable gastric band 10 by suturing portion 14 to the stomach. Referring also to Fig. 2, the adjustable gastric band 10 includes a non-extensible strap 16 and an inflatable balloon 18, made of a medical grade silicone polymer or any other suitable material, is carried by the inner surface 20 of the strap 16. The balloon 18 may be secured to the inner surface 20 in any well known manner, or even made of unitary construction with the strap 16, although the strap 16 may typically be formed of a different material.

One end of a flexible conduit 22 is in fluid communication with the internal cavity 24 of the balloon 18, with the other end being in fluid communication with an internal cavity (not shown) of a remote injection port 26. The remote injection port 26 includes a silicone septum 28. At the time the adjustable gastric band 10 is implanted around a portion of the stomach, the remote injection port 26 is also implanted at a suitable location, usually within the rectus sheaths, for transcutaneous access via a Huber needle.

As is well known, the internal cavity 24, the flexible conduit 22 and the internal cavity of the remote injection port 26 are preferably at least partially filled with a physiologically compatible fluid, such as a saline solution. Postoperative adjustment of the perimeter enclosed by the balloon 18, and therefore the size of the stoma, is accomplished by addition or removal of fluid from the interior cavity 24 of the balloon 18 by inserting a Huber needle percutaneously into the silicone septum 28 of the injection port 18.

As is well known in the field the adjustable gastric band 10 may be made from any suitable medically compatible material having sufficient strength necessary for a particular laparoscopic surgery or particular patient.

As mentioned above, the two ends of the adjustable gastric band 10 are attached together (the specific attachment mechanism structure is not illustrated in detail in FIG. 1).

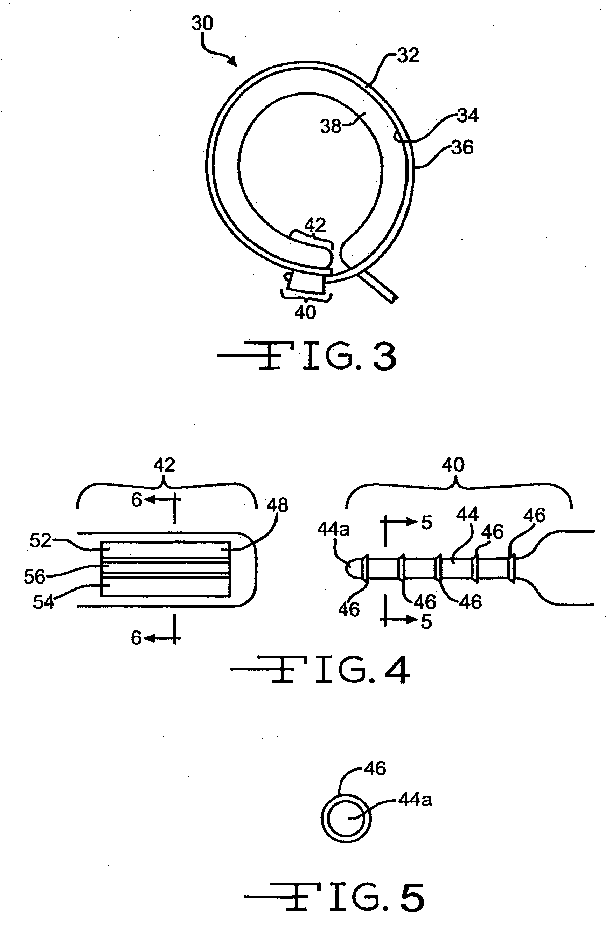

The present invention is directed to various embodiments of attachment mechanisms, for connecting the two ends together, which comprise a material that has at least one material property which different from that of the material of which the band is primarily comprised. The general construction of adjustable gastric band 10 shown in FIGS. 1 and 2 and described above is common to the embodiments illustrated in FIGS. 3-7, with the embodiments differing by the specific attachment mechanisms. It is noted that the practice of the present invention may be used with any band, and is not limited to use with an adjustable gastric band having the exact features described above or below.

Turning now to FIG. 3, the adjustable gastric band 30 includes an elongated strap 32 extending in what is referred to herein as the longitudinal direction, even though when implanted the adjustable gastric band 30 has an arcuate configuration. The strap 32 includes an inner surface 34 and an outer surface 36, with the balloon 38 extending inwardly from adjacent the inner surface 34. The adjustable gastric band 30 includes a first end portion 40 which overlaps a second end portion 42. The first and second end portions 40, 42 are secured together by a deformable attachment mechanism.

Referring also to FIG. 4, in the embodiment depicted, the first end portion 40 of strap 32 has a first portion of the attachment mechanism associated with it depicted as an elongated cylindrical shaft portion 44 having a plurality of engagement members 46, shown as frustroconical annular flanges 46, axially spaced along the shaft portion 44, angled away from the distal end 44a. The second end portion 42 of strap 32 has a second portion of the attachment mechanism associated with it depicted as a laterally extending member 48 which extends generally perpendicularly from the outer surface 36.

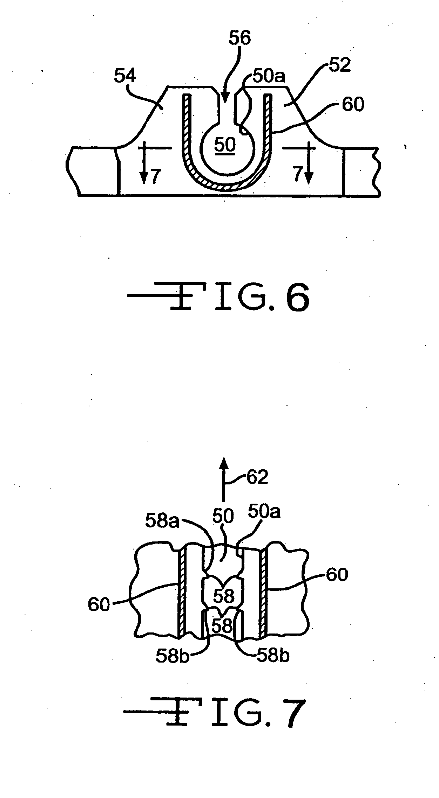

The member 48 is configured to receive the first end portion 40. Referring also to FIGS. 6 and 7, the laterally extending member 48 at least partially defines a cavity, also referred to as a passageway, 50 which is configured to receive the first end portion 40. The member 48 includes two spaced apart legs 52, 54, which extend from the outer surface 36, and define a gap 56 therebetween. Gap 56 leads to the passageway 50.

As seen in FIG. 7, the surface 50a of passageway 50 is configured to cooperate with the engagement members 46 to resist axial withdrawal of the first end portion 40 from the passageway 50. More specifically, passageway 50 includes a plurality of axially spaced, generally annular shaped retention members 58, also referred to as rings 58, whose annular shape is interrupted by gap 56. In the embodiment depicted, each retention member 58 includes a generally frustroconical surface 58a and a generally transverse (to the longitudinal direction) retention surface 58b.

The passageway 50 and a portion of gap 56 is surrounded by a retention actuator 60, shown as a "U" shaped member although any suitable shape may be used, disposed completely within the member 48. The actuator 60 is made of any material which may be easily plastically deformed, or crimped to the desired shape without breaking, so as to urge the retention members 58 against the shaft 44 to produce increased resistance to withdrawal, axially or laterally, of the first end portion 40. Thus, at least this one material property of the actuator 60, plastic deformability which is not preceded by significant elastic deformation, a relatively low yield point, is different from the corresponding material property of the material from which the rest of the strap 16 is made. Energy is imparted to the actuator 60 to plastically deform it, resulting in secure attachment of the first end portion 40 to the second end portion 42.

More specifically, crimping deforms the actuator 60 causing it to squeeze against the shaft 44, causing the retention members 58 to present greater resistance to axial movement of the engagement members 46 in the direction of the arrow 62. Crimping may also close the gap 56.

To attach the first end portion 40 to the second end portion 42, the shaft 44 is pushed through the passageway 50. It is possible with the embodiment illustrated to set the band 30 to more than one diameter by advancing the shaft 44 through the passageway 50 to a desired position, with the engagement members 46 meeting minimal resistance to such axial movement from the frustroconical surfaces 58a. Once in the desired position, the actuator member 60 can actuate the attachment mechanism by being crimped with a grasper or other suitable device. To detach the two ends, the closed gap 56 may be spread apart such as by spreading the opposing surfaces of the gap 56 apart with a grasper.

The actuator 60 may be made of any biologically suitable material which provides the desired deformation and force when crimped, such as a plastically deformable metal. Although the actuator 60 is shown completely disposed within the second end portion 42, such as insert molded, the actuator 60 may extend beyond the exterior surface so long as it remains retained to the second end portion 42 in some manner, and the integrity of the second end portion 42 is preserved. The width of gap 56 is selected to provide sufficient clearance to lay the shaft 44 therethrough and to be closed by crimping so that deformation of the actuator 60 is not unduly limited.

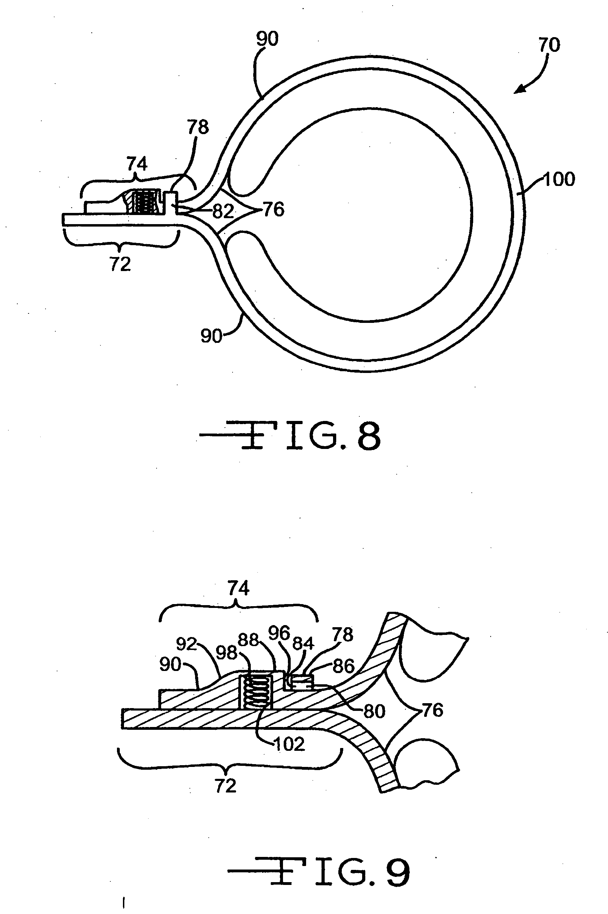

Referring now to FIGS. 8 and 9, another embodiment is illustrated having a deformable attachment mechanism which is elastically deformable. The adjustable gastric band 70 includes a first end portion 72 and a second end portion 74, shown attached together with the inner surfaces 76 at each end 72 and 74 abutting each other. The first end portion 72 includes a first portion of the attachment mechanism associated with it depicted as a laterally extending member 78 which extends generally perpendicularly from the inner surface 76 of end 72. The member 78 is configured to engage the second portion of the attachment mechanism associated with the second end portion 74. More specifically, the laterally extending member 78 defines a passageway 80 in conjunction with the inner surface 76 at the first end portion 72 which is configured to receive the second end portion 74. The member 78 includes two spaced apart legs 82 and 84 which extend from the inner surface 76 at the opposite edges of the strap 70, with a cross member 86 extending therebetween.

The second portion of the attachment mechanism associated with the second end portion 74 is depicted as including a retaining member 88 extending laterally from the outer surface 90 thereof. The retaining member 88 includes an inclined surface 92 which may be arcuate as shown, or planar. The retaining member 88 also includes the retention surface 96 which extends generally perpendicular from the outer surface 90, and perpendicular to any relative movement between the first and second end portions 72 and 74. The retention surface 96 must extend above the upper edge of the passageway 80 a distance sufficient to provide the desired force to resist disengagement. In the embodiment depicted, the retention surface 96 extends slightly beyond the top of the cross member 86.

The retaining member 88 includes a member 98, preferably made of a dissimilar material, or at least having dissimilar resilience properties, from the rest of strap 100. In particular, in this embodiment, the elasticity of the material from which member 98 is made is different than the elasticity of the material from which the strap 100 is made, having greater elasticity so as to bias the retaining member resiliently toward the shape shown. With the configuration illustrated, the greater elasticity allows easier compression of the retaining member 88 when inserting it through the passageway 80.

In the embodiment disclosed, the member 98 is diagrammatically shown as a coil spring, disposed in a cavity 102 formed in retention member 88. The cavity 102 with the member 98 may be formed using any suitable method, such as a two step molding process, insert molding, or other known techniques. The presence of cavity 102 allows retention member 88 to deflect in the lateral direction more easily than if the cavity was the same material as the rest of the band 100, typically silicone. The member 98 is laterally resilient, urging the retention member 88 outwardly so as to maintain the retention surface 96 in a position that requires high longitudinal force to separate the two ends. With the laterally resiliency of member 98, perpendicular to the direction of removal, and the cavity 102, only a relatively light lateral force is required to move the retention surface 96 below or nearly below the cross member 86 to a position that does not block or presents reduced resistance to withdrawal of the second end portion 74 from the passageway 80.

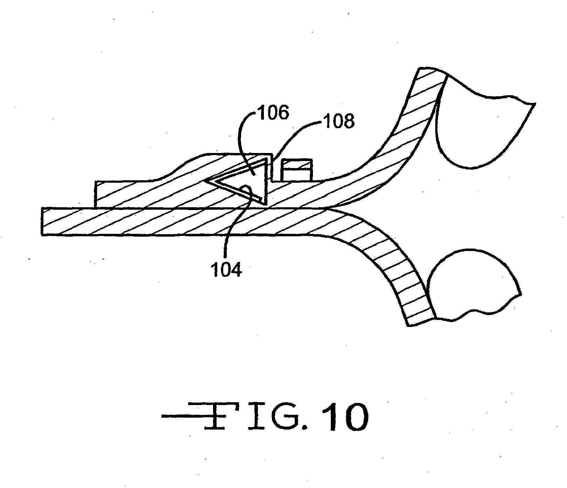

The dissimilar material, elastically deformable element and cavity within which it is disposed may be of any suitable shape. Referring to FIG. 10, an another embodiment of a resilient member 104 is diagrammatically illustrated disposed in the cavity 106. The member 104 has a "V" shape, with the wide end opening toward the retention surface 108.

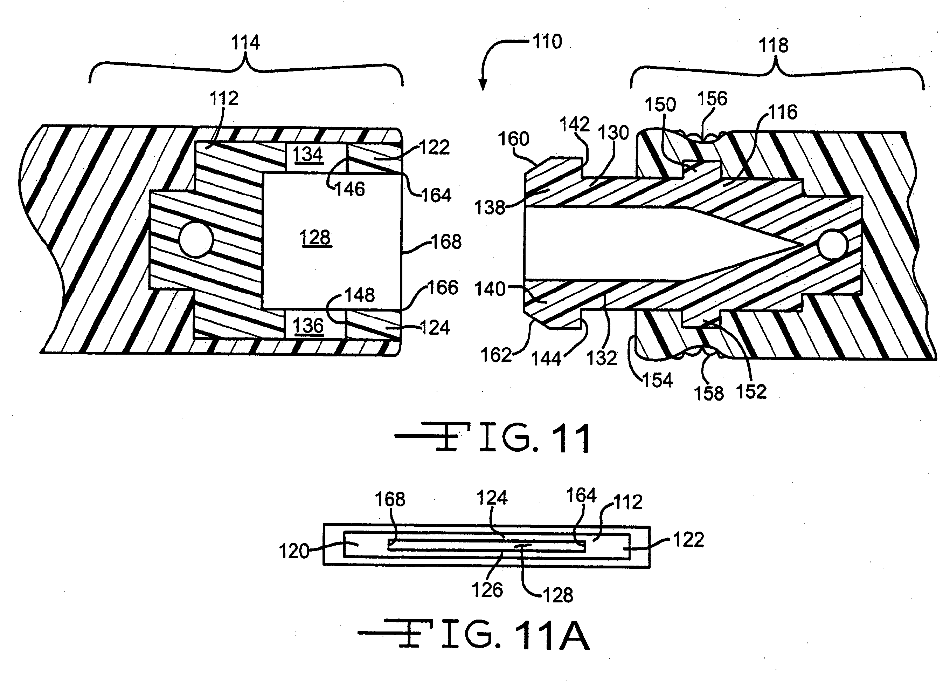

Referring to FIG. 11, there is shown another embodiment of an attachment mechanism, generally indicated at 110, which includes a receiver 112 carried by the first end portion 114, and a resilient member, illustrated as a dual cantilever spring 116 carried by the second end portion 118. The receiver 112 and the spring 116 may be made of any suitable medically compatible material having sufficient strength. In the embodiment depicted, the receiver 112 and the spring 116 are made of an injection moldable polymer which are insert molded into the first and second end portions 114 and 116 of the adjustable gastric band (not numbered in FIG. 11), which is made of silicon. The material properties of the material from which the receiver 112 and the spring 116 different from the material properties of the material from which the strap is made. In particular in this embodiment as illustrated, the material is stiffer, more rigid and harder than the material (e.g., silicone) of the strap.

Referring also to FIG. 11A. the receiver 112 includes two pairs of spaced apart side walls 120, 122, 124 and 126 which defines a cavity, also referred to as a pocket, 120 that is configured to receive the two spaced apart legs 130 and 132 of the spring 116. The side walls 122 and 124 include two openings 134 and 136 which are configured to receive the ends 138 and 140 of the legs 122 and 124. Each end 138 and 140 include a respective step 142 and 144 which is dimensioned to respectively engage the side 146 and 148 of the respective opening 134 and 136 to resist withdrawal from the pocket 128, as described below.

The spring 116 includes steps 150 and 152 extending outwardly from a respective leg 138 and 140. The second end portion 118 is molded about a portion of the spring 116 retaining it thereto, with the legs 138 and 140 extending from the end 154. There are recessed surfaces 156 and 158, which may include surface texturing or protuberances as illustrated, generally aligned with the steps 150 and 152, providing a location to grasp the second end portion 118 to squeeze the ends 138 and 140 toward each other.

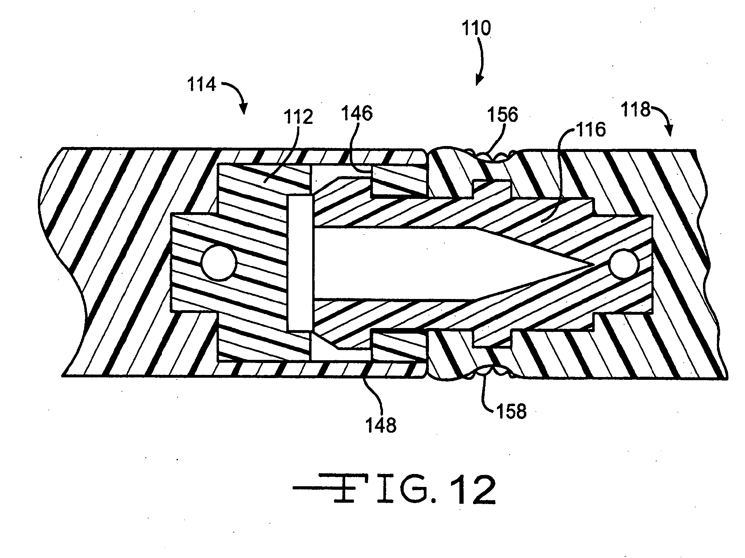

Referring also to FIG. 12, to connect the attachment mechanism 110 together, the legs 130 and 132 are inserted into the pocket 128. The ends 138 and 140 includes ramps 160 and 162 which engage comers 164 and 166 upon insertion, compressing the legs 130 and 132 toward each other as the legs are advanced into the pocket 128. The material and construction of the spring 116 provides resiliency to the two legs 138 and 140. The ends 138 and 140 are advanced into the pocket 128 until they can move outwardly to snap into the openings 134 and 136 and hold the two end portions 114 and 118 together with end 154 generally abutting end 168.

As depicted, the openings 134 and 136 extend completely through the spaced apart side walls 122 and 124, respectively, of the receiver 112 and the steps 142 and 144 do not extend outside of the openings. The openings 134 and 136 are depicted as being covered by the silicon material of the end portion 114. Once attached as show, the receiver 112 and spring 116 are not exposed, enclosed within the band shroud, posing no erosion threat to the surrounding tissue.

To detach the first end portion 114 from the second end portion 118, the recessed surfaces 156 and 158 are squeezed inwardly, causing the legs 130 and 132 to move inwardly until the ends 138 and 140 withdraw from the openings 134 and 136, and the spring 116 can be withdrawn from the receiver 112. The recessed surfaces 156 and 158 may be squeezed by use of a grasper.

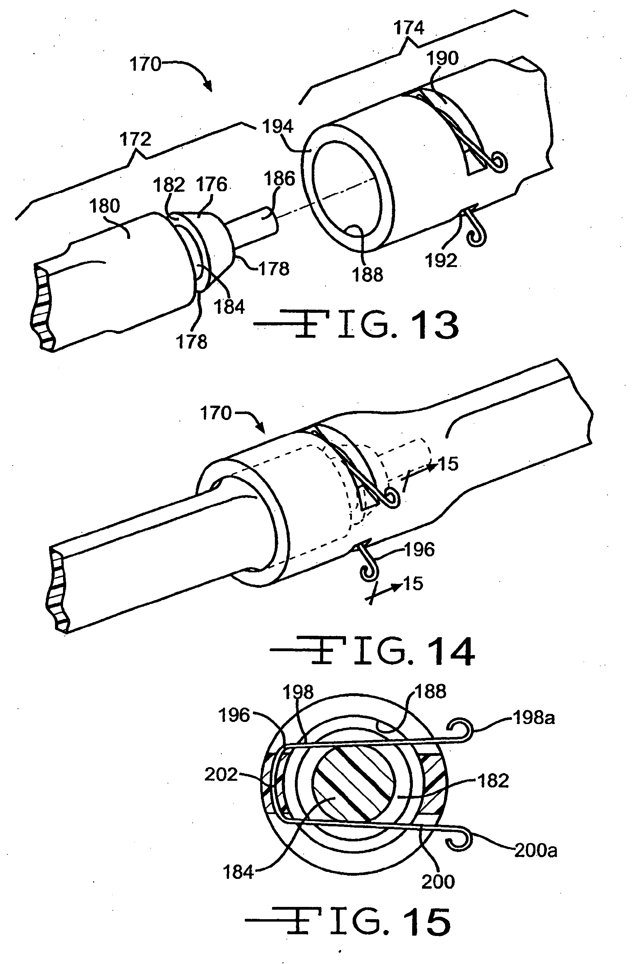

Referring to FIGS. 13-15, there is shown yet another embodiment of an attachment mechanism, indicated generally at 170 which includes a first end portion 172 and a second end portion 174 made of the same material as the rest of the band, such as silicon. The first end portion 172 includes a conically shaped retention member 176, more specifically illustrated as a frustroconical shape, disposed adjacent an annular groove 178 separating a first cylindrical portion 180 and the base 182 of retention member 176. A second cylindrical portion 184 extends between the first cylindrical portion and the retention member 176. Extending from the opposite end of retention member 176 is another cylindrical portion 186.

The second end portion 174 defines an internal, generally cylindrical cavity 188, also referred to as an opening, which is shaped complementarily to and configured to receive the first end portion 172, as depicted having an inner diameter slightly larger than the outer diameter of the first cylindrical portion 180 by an amount sufficient to allow the first end portion 172 to be inserted into the cavity 188 in the manner described below. Second end portion 174 includes two spaced apart transverse slot openings 190 and 192 in communication with the internal cavity 188, disposed on opposite sides of the second end portion 174.

A resilient member 196, depicted as a generally U shaped spring clip having two legs 198 and 200 extending from a base 202, generally parallel to each other in the free state. The spring clip 196 may be made of any suitable medically compatible material providing the necessary resilience and strength. One or more material properties of the material from which the spring clip 196 is formed is different than the corresponding one or more material properties of the material from which the strap is made. In the depicted embodiment, the spring clip 196 has more hardness, rigidity, stiffness, resiliency and elasticity than the strap.

The spring clip 196 is carried by the second end portion 174 with the legs 198 and 200 disposed partially in the openings 190 and 192, respectively. The end portions of the legs 198a and 200a extend beyond the openings 190 and 192, and terminate in curved portions. The base 202 is molded into the second end portion 174, leaving the legs 198 and 200 free to be spread outwardly to allow retention member 176 to pass between the legs 198 and 200.

In the free state, the distance between the legs 198 and 200 is sufficient to permit the leading end 176a of retention member 176 to pass therebetween. Preferably, the diameter of end 176a is smaller than the corresponding distance between the legs 198 and 200. To connect the attachment mechanism 170 together, the first end portion 172 is inserted into the cavity 188. The inclined shape of the retention member 176 spreads the legs 198 and 200 apart as the first end portion 172 is advanced into the cavity 188, until the openings 190 and 192 are aligned with groove 178, whereat the legs 198 and 200 move into the groove 178, preferably being resiliently urged against the cylindrical portion 184 as seen in FIG. 15, but at least move to a position sufficient to retain the first end portion 172 within the cavity 188 adjacent the base 182 of retention member 176.

To detach the first end portion 172 from the second end portion, the legs 198 and 200 are pulled apart far enough to allow the base 182 to pass therebetween. This may be accomplished by use of a grasper.

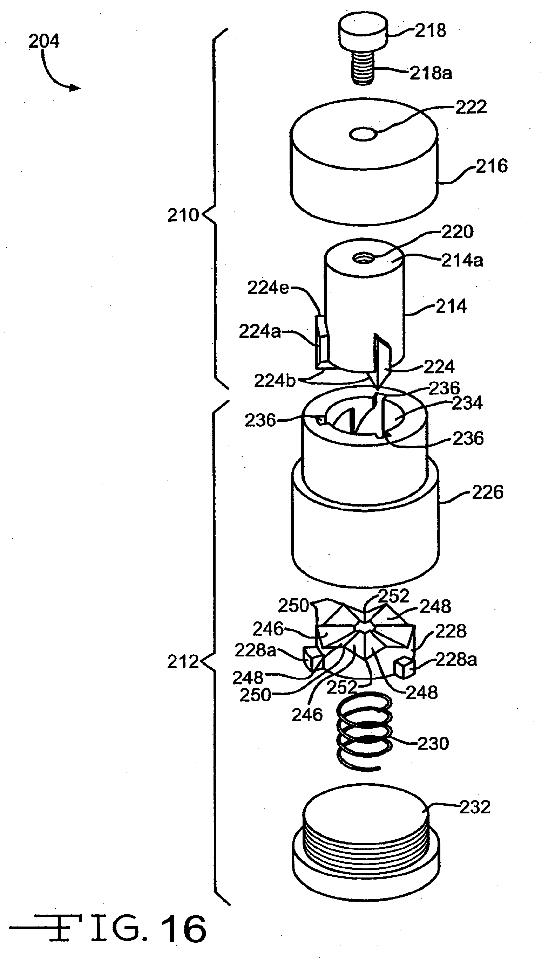

Referring generally to FIGS. 16-23, and in particular to FIG. 16 and FIGS. 21-23, there is shown another embodiment of an attachment mechanism, indicated generally at 204, of an adjustable gastric band 254, which includes a first end portion 206 and a second end portion 174. FIG. 16 illustrates the attachment mechanism 204, without the band, in an exploded perspective view. The first end portion 206 carries the insert assembly 210 and the second end portion 208 carries the housing assembly 212. The insert assembly 210 and housing assembly 212 may be attached to the first and second end portions 206 and 208, in any suitable manner. In the depicted embodiment, insert assembly 210 and housing assembly 212 have been molded into the end portions 206 and 206 respectively, aligned longitudinally with the band 254. It is noted that the insert assembly 210 and housing assembly 212 could alternatively be oriented lateral or transverse to the band 254.

In the depicted embodiment, the insert assembly 210 includes the insert 214, a cap 216 and a retainer 218. The insert 214 is rotatably connected to the cap 216 by retainer 218. As depicted, the insert 214 includes a hole 220 which is configured to receive part of the retainer 218, which is illustrated as a threaded pin, although any suitable retainer or retaining structure may be used. The cap 216 also includes a hole 222 through which the threaded shaft portion 218a of retainer 218 extends to engage with the internal threads of hole 220. The cap 216 may include a counterbore or other recess (not shown) in its surface disposed adjacent the upper end 214a of the insert 214. Such a recess may be shaped complementarily to the adjacent portion of insert 214 so as to receive a portion of insert 214 while still allowing sufficient rotation of insert 214. Any configuration which allows an insert to be carried suitably freely rotating by the first end portion 206 may be used.

The insert 214 includes three legs 224, each of which includes a radially extending portion 224a and an axially extending portion 224b. Although three spaced legs 224 are depicted, there may be one or more spaced apart legs. In the embodiment depicted, legs 224 are equally circumferentially spaced, having 120° angles between them. Although the axially extending portions 224b are continuous and aligned with the radially extending portions 224a, the function of the legs 224 can be achieved without being continuous and aligned.

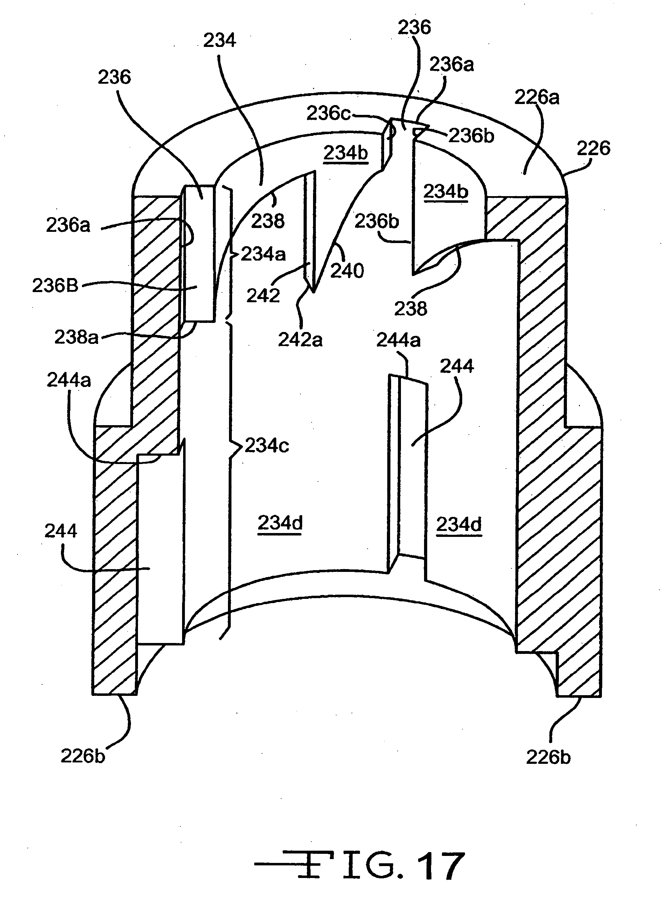

In the depicted embodiment, the housing assembly 212 includes the housing 226, the resistor 228, biasing member 230, depicted as an elastically deformable coil spring, and end cap 232. Referring also to FIG. 17, the housing 226 defines an internal cavity, also referred to as a bore, 234. The first portion 234a, also referred to as the entrance portion, of bore 234, starting at the upper end 226a of housing 226, has a nominal diameter which is complementary to the nominal outer diameter (not including the legs 224) of the insert 214. A plurality of longitudinal slots 236 are formed extending outwardly from the inner surface 234b of the entrance portion 234a. The slots 236 are configured to receive the legs 224, the number and spacing of slots 236 matching the number and spacing of the legs 224. The width (circumferentially) and the depth (beyond the inner surface 234b) of the slots 236 are sized to provide clearance for the legs 224 to slide axially therethrough with no or little axial force required. As will be described, since the insert 214 rotates within the housing 226 to connect and disconnect (actuate and deactuate) the attachment mechanism 204, it is preferable that the legs 224 and the slots 236 be equally and uniformly spaced, circumferentially/angularly.

The bore 234 includes a second portion 234c. The second portion 234c has a diameter provides diametrical clearance for the legs 224 so that the insert 214 may freely rotate within the housing 226 once the legs 224 have cleared the slots 236, as will be described below. In the depicted embodiment, the diameter of the second portion 234c is substantially the same as the depth of the slots 236, with the inner surface 234d being continuous with the bottoms 236a of slots 236.

The entrance portion 234 defines a plurality of arcuate ramps 238 and 240 which extend outwardly relative to the inner surface 234b a distance that provides diametrical clearance for the legs 224. The ramp 238 extends between the lower end of the longitudinally extending side 236b to the upper end of the longitudinally extending stop surface 242. The ramp 240 extends between the lower end of the stop surface 242 to the lower end of the longitudinally extending side 236c. In the depicted embodiment, the inner surface 234d extends to the ramps 238 and 240.

A plurality of longitudinal slots 244 are formed extending outwardly from the inner surface 234d from the lower end of second portion 234c. The slots 244 are configured to receive the legs 228a of the resistor 228, providing enough clearance for the legs 228a to slide axially slide axially therealong with no or little axial force required, until stopped by the ends 244a. The number and spacing of slots 244 match the number and spacing of the legs 228. In the depicted embodiment, the grooves 244 and the legs 228 are equally and uniformly spaced, circumferentially/angularly, although any number and orientation which provide the desired function may be used.

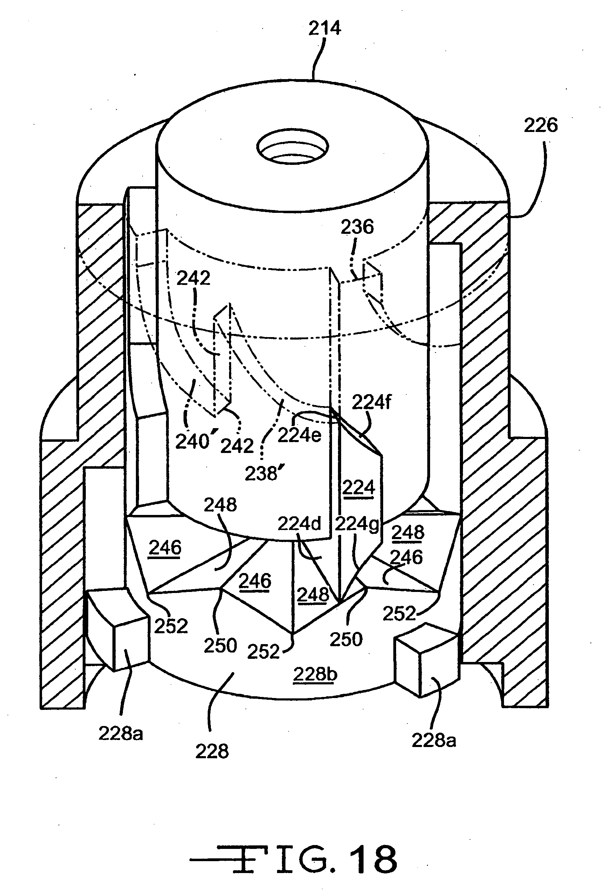

Referring also to FIG. 18, the inner surface 234d is shaped complementarily to the outer surface 228b of the resistor 228 which is disposed in the bore 234. In the depicted embodiment, the surface 228b is cylindrical and has a diameter which is smaller than the diameter of the inner surface 234d by an amount sufficient to allow the resistor 228 to slide axially therein with no or little axial force required. To retain the resistor 228 within the housing 226, the end cap 232 is secured to the lower end 226b, with biasing member 230 disposed between the lower end of the resistor 228 and the upper end of the cap 232, urging the resistor 228 toward the entrance end 234a. The cap 232 and the lower end 226b may be connected together in any suitable manner, such as threads.

The resistor 228 includes a plurality of inclined ramps 246 and declined ramps 248 (in the clockwise direction when viewed from the top) which intersect at radially oriented peaks 250 and valleys 252. The ramps 246 and 248, and the axially extending portion 224b are configured to cooperate together to bias the insert 214 rotationally as the insert 214 is urged against the resistor 228 during actuation and deactuation of the attachment mechanism 204. In the depicted embodiment, the axially extending portions 224b each include a lower surface 224c which terminates in a radially oriented edge 224d which is configured to engage the ramps 246 and 248 of the resistor 228. As will be appreciated, the configurations of the ramps 246 and 248, the axially extending portions 224b, the surfaces 224c and the edges 224d must be complementary to each other at the smaller circumferential distances approaching the center of the end of the insert 214, to avoid interference. Other configurations may be used which achieve the same functionality of these features. For example, the axially extending portions 224b may extend only axially, aligned with the rest of legs 224, not radially inward beyond the circumference of the insert 214.

FIGS. 18-20 illustrate various positions of the insert 214 during actuation and deactuation of the attachment mechanism 204. It is noted that in FIGS. 18-20, the curvature of ramps 238' is opposite that of the curvature illustrated in FIG. 17. The shapes of the ramps and the legs may be any suitable shapes which cooperate together to achieve the indexed rotation of the insert 214 as described below.

In FIG. 18, the insert 214 is illustrated disposed at least partially in the bore 234 of the housing 216, with each leg 224 being disposed within a respective slot 236. When the legs 224 are so located, each edge 224d of the axially extending portions 224b is disposed to engage respective declined ramps 248. When the insert 214 is advanced axially into the bore, the declined surfaces of the ramps 248 bias the edges 224d rotationally, but rotation is initially prevented by stop 236b, until the insert 214 has been advanced far enough into the bore 234 for the upper edges 224e to clear the lower ends 238a' of the sides 236b/lower end of the ramps 238'. The resistor 228 moves axially within bore 134 as the insert 214 advances, but does not rotate.

Once the upper edges 224e have cleared the lower ends 238a', and the insert 214 can rotate, the declined surfaces of the ramps 248 cause the insert to rotate until the lower edges 224d reach the valleys 252, which are the terminuses of the declined surfaces 248-the portions of the declined surfaces 248 at which rotation of the insert ceases. At this location, the upper edges 224e have rotated past the lower ends 238a', and underlie the ramps 238'.

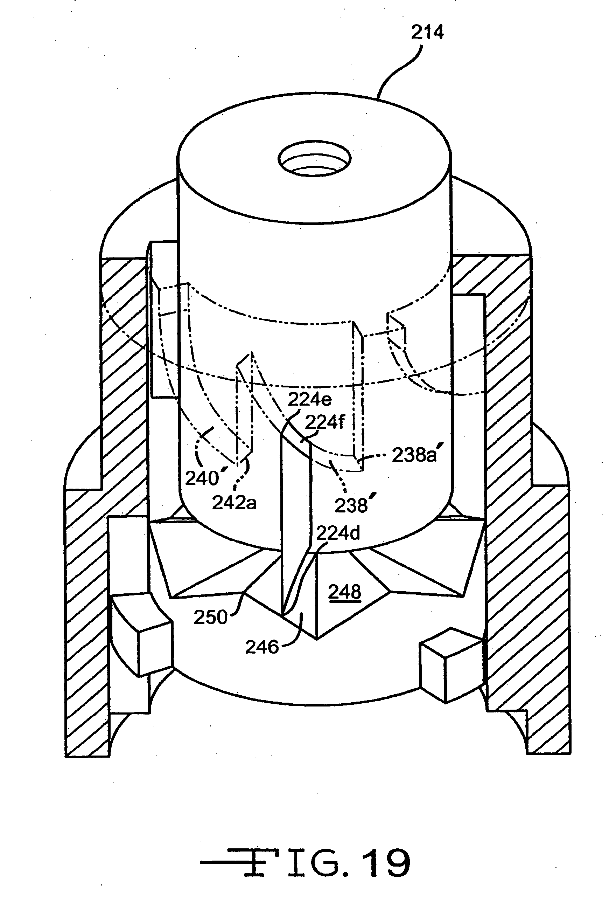

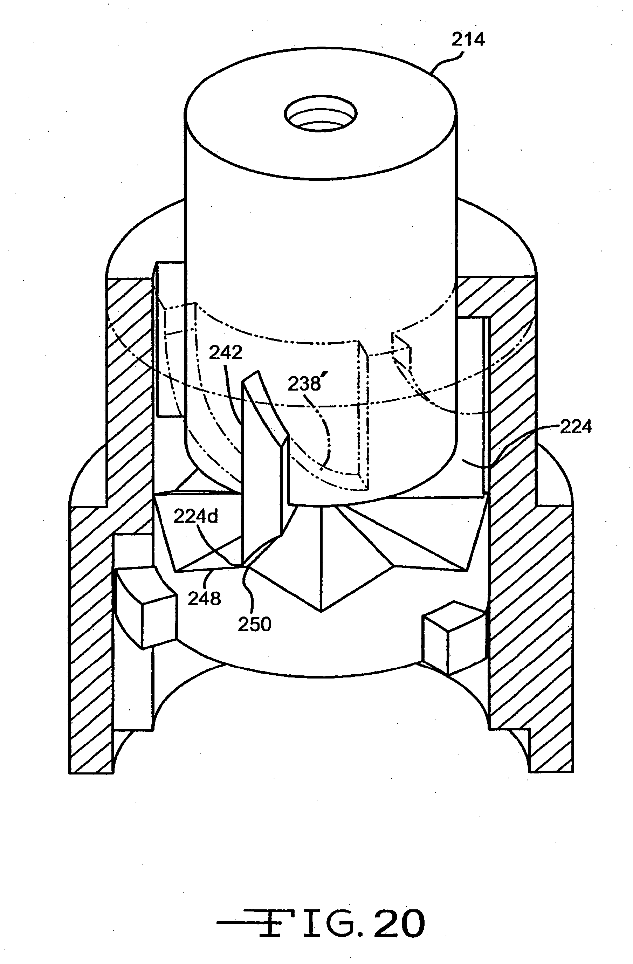

When the axial force on insert 214 is then released, the upwardly biased resistor 228 urges the insert 214 upwardly, through the contact of edges 224d with the inclined ramps 246, urging the upper edges 224e into engagement with the ramps 238'. It is noted that the upper edges 224e may be the only part of the radially extending portion 224a that contacts the surfaces 238', as illustrated in FIG. 18, or the surfaces 224f may be configured to engage the ramps 238' in addition to engagement by the edges 224e, as illustrated in FIGS. 19 and 20, or to engage the ramps 238' instead of the upper edges 224e.

FIG. 19 illustrates the engagement of the upper edges 224e and surfaces 224f with the ramps 238', which exert an advancing (clockwise in the illustration) rotational bias on the insert 214. Concomitant with the upward biasing by the resistor 228, the inclined ramps 246 resist the advancing rotation of the insert 214. However, the advancing rotational bias imparted by ramp 238' is sufficient to overcome the resisting rotational bias imparted by the inclined ramps 246, and the insert 214 advances rotationally until rotation is stopped by stop surface 242, as illustrated in FIG 20, and the insert is retained in the bore 234 and the attachment mechanism 204 is actuated, securing the ends of the band together.

As seen in FIG. 20, the lower edges 224d have advanced beyond the peaks 250. To separate the ends of the band, the attachment mechanism 204 is deactuated by depressing the insert 214, i.e., advancing the insert into the bore 234, urging the lower edges 224d against the declined ramp 248. As with the actuation process, the declined ramps 248 bias the edges 224 rotationally, but in this case rotation is initially prevented by the stop surface 242, until the insert 214 has been advanced far enough into the bore 234 for the upper edges 224e to clear the lower ends 242a of the stop surface 242.

Once the upper edges 224e have cleared the lower ends 242a, and the insert 214 can rotate, the declined surfaces of the ramps 248 cause the insert to rotate until the lower edges 224d reach the valleys 252. At this location, the upper edges 224e have rotated past the lower ends 242a, and underlie the ramps 240'.

When the axial force on insert 214 is then released, the upwardly biased resistor 228 urges the insert 214 upwardly, through the contact of edges 224d with the inclined ramps 246, urging the upper edges 224e into engagement with the ramps 240'. As mentioned above in reference to surfaces 238', the upper edges 224e may be the only part of the radially extending portion 224a that contacts the surfaces 240', or the surfaces 224f may be configured to engage the ramps 240' in addition to engagement by the edges 224e or to engage the ramps 238' instead of the upper edges 224e.

The engagement of the upper edges 224e and surfaces 224f with the ramps 240' exert an advancing rotational bias on the insert 214, with the inclined ramps 246 resisting the advancing rotation of the insert 214. The advancing rotational bias imparted by ramp 240' is sufficient to overcome the resisting rotational bias imparted by the inclined ramps 246, and the insert 214 advances rotationally into the slots 236, allowing the insert 214 to be withdrawn.

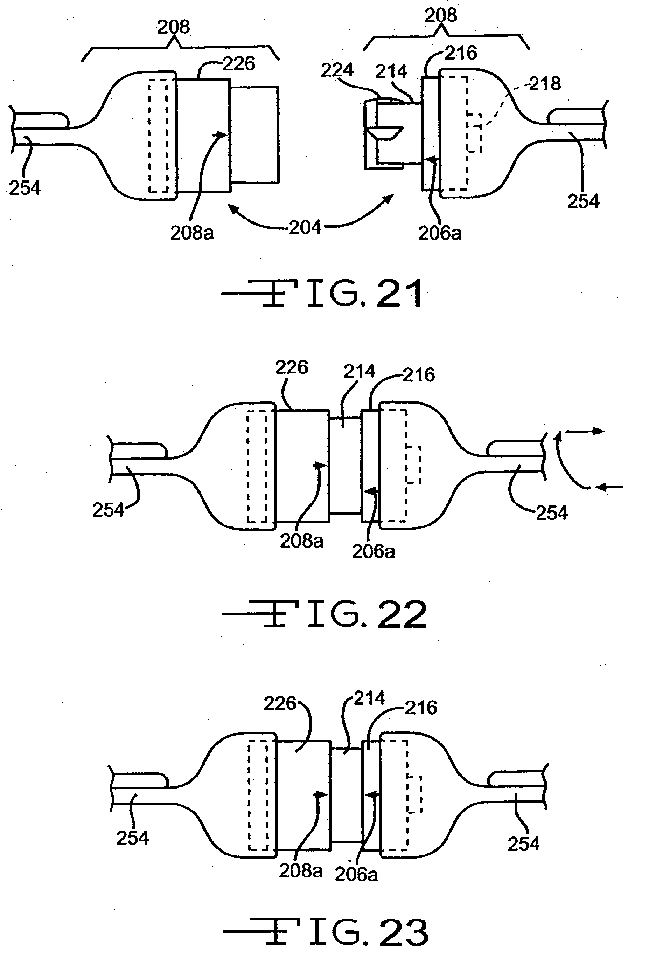

Referring to FIGS. 21-23, the attachment mechanism 204 is illustrated being actuated. FIG. 21 illustrates the first and second end portions 206 and 206 prior to actuation, generally aligned and proximal to each other. There are arrows 206a and 208a shown on the cap 216 and the housing 226 in these figures to illustrate the rotation of the insert 214 during actuation, and may be, but not necessarily, included in the actual device to allow visualization of the relative orientation and of rotation of the insert 214 during actuation. The legs 224 are aligned with the slots 236 so that the insert 214 may be inserted into the bore 234. FIG. 22 illustrates the insert 214 advanced into the bore 236, a position at which the resistor 228 has moved axially. The insert 214 is caused to rotate as it is inserted far enough, and upon release of the axial force, is retained in the housing 226 as shown in FIG. 23, with the arrows 206a and 208a aligned. Deactuation is accomplished by advancing the insert 214 axially so it rotates, and then withdrawing it from the bore 234.

The components of the attachment mechanism 204 may made of any medically compatible materials, such as but not limited to metal, plastic or a combination thereof. In the embodiment depicted, the attachment mechanism 204 is made of different material(s) than the band. The material properties of the material(s) from which the insert assembly 210 and the housing assembly 212 are made are different from the material properties of the material from which the strap is made. Generally, the components are stiffer, more rigid and harder. The biasing member 230 has greater elasticity and resiliency.

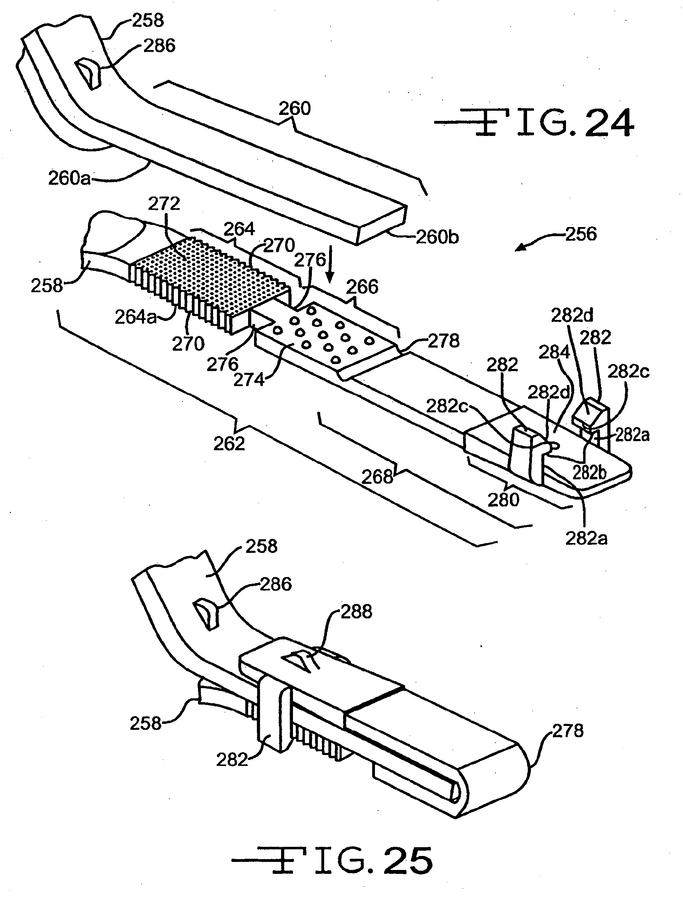

Referring to FIGS 24-26, there is shown another embodiment of an attachment mechanism, indicated generally at 256, of an adjustable gastric band 258, which includes a first portion of the attachment mechanism associated with the first end portion 260 and a first portion of the attachment mechanism associated with the second end portion 262. The first end portion 260, also referred to as a tongue portion, may be formed of the same material as the band 258, and is depicted as generally having the same width and thickness as the band 258.

In the embodiment depicted, the second portion 262 includes a proximal section 264, which is proximal to the band 258, an intermediate section 266, and a distal section 268. The edges 270 of the proximal section 264 are roughened or textured in order to resist unintended separation of the attachment mechanism 256, as described below. The edges 270 are depicted as including a plurality of laterally oriented ridges along the length of the proximal section 264. The edges 270 may be roughened or textured along their entire lengths, as depicted, but are not required to be. The inner surface 272 of the proximal section 264 is depicted as textured, but it may alternatively not be textured.

The intermediate section 266 includes a textured surface 274 which is configured to resist, and preferably prevent, relative longitudinal movement between the first end portion 260 and the second end portion 262 when the attachment mechanism 256 is actuated, as described below. A pair of outwardly opening recesses 276, also referred to as release slots, may be formed in the intermediate section 266 adjacent the proximal section 264. The recesses 276 may alternatively characterized as being be disposed adjacent the intermediate section 266 and the proximal section 264, as disposed between the intermediate and proximal sections, as disposed in the proximal section 264 adjacent the intermediate section 266, or as disposed in both the proximal and intermediate sections. It is the location of the recess 276 relative to the latches (described below) which is relevant to the operation of the attachment mechanism 256, not such characterization.

The distal section 268 is pivotable relative to the intermediate section 266 through the hinge 278. Depending on the material, the hinge 278 may be an elastomeric hinge or a plastic living hinge.

The distal end 280 of the distal section 268 includes two spaced apart latches 282 extending laterally from the edges of the distal end 280. As depicted, the latches 282 define respective upright members having inwardly facing surfaces 282a which are preferably spaced apart a distance less than the distance between the edges 270. The distal end 280 may be tapered, the width of distal end 280 decreasing along its length, or have a smaller width along its length in comparison to the width between the edges 270, such that the inner surfaces 282a engage the edges 270. The width of the first end portion 260, adjacent the latches 282 when the attachment mechanism 256 is attached, the width between the edges 270 and the width between surfaces 282a are configured to allow the surfaces 282a to engage the edges 270.

The latches 282 defined inwardly extending surfaces 282b which overlie the inner surface 284 of the distal end 280. The longitudinal widths of the latches 282, and the transverse distance between the inner surfaces 282c of the latches 282 allow the latches 282 to pass through the recesses 276 without significant resistance, as described below.

Referring to FIG. 25, the inner surface 260a of the first end portion 260 is disposed adjacent the proximal section 264 and the intermediate section 266, with the distal end 260b adjacent the hinge 278. The distal section 268 is pivoted about the hinge 278 to capture the first end portion 260 between the distal section 268 and the proximal and intermediate sections 264 and 268. The distal section 268 exerts a clamping force against the first end portion to urge the inner surface 260a proximal to the distal end 260b against the textured surface 274 between the first end portion 260 and the second end portion 262, which may result at least in part due to the dimensions of the hinge 278. The inclined surfaces 282d of the latches 282 act to spread the latches 282 apart allowing the latches 282 to be pushed downwardly past the first end portion 260 and the proximal section 264, until the inner surfaces 282c have cleared the edges 270, allowing the inwardly facing surfaces 282a to engage edges 270 and the inwardly extending surfaces 282b to abut or at least face the outer transverse surfaces 264a, in the latched position as shown in FIG. 25.

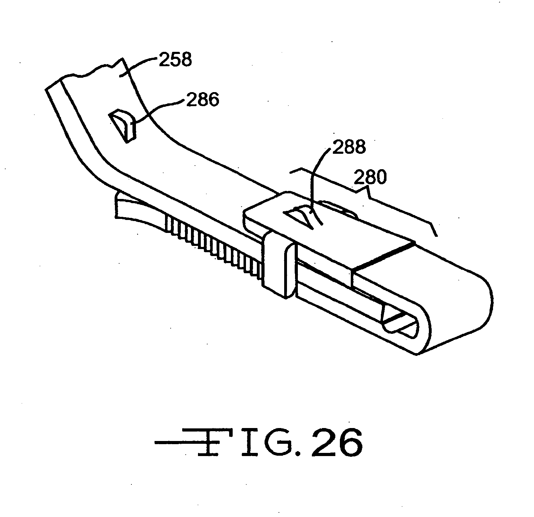

FIG. 26 illustrates the attachment mechanism in an intermediate stage of being disconnected. To deactuate or disconnect the attachment mechanism 256, the distal end is moved longitudinally relative to the proximal section 264 and intermediate section 266, until the latches 282 are aligned with the recesses 276, allowing the distal ends of the latches 282 to be moved laterally into the recesses 276 allowing the proximal and intermediate sections 264 and 266 to pivot relative to the distal section 268, thereby reducing the clamping force against the first end portion 260. Once the clamping force has been reduced, the first end portion may be separated from the second end portion 262.

The outer surfaces of the band 258 include pull tabs 286 and 288 which may be grasped using a grasper or other suitable instrument to effect the movement of the distal end 280.

As mentioned above, the first end portion 260 may be made of the same material as the band 258, which is typically silicon. The distal end 280 may be made of any material having sufficiently rigid and elastic material properties to provide the necessary resiliency to the latches 282 and to produce the necessary clamping load to retain the first end portion 260, such as a hard plastic material, and thus is made of a material which is different from the rest of the band 258. The entire second end portion 262 may be, but is not necessarily made from a rigid material. Alternatively, any sections up to the distal end 280, or possibly even up to the latches 282, may be made of the same material or a material with similar properties as the material of the rest of the band 258. For example, all of distal section 268 may be made of a rigid material. The hinge 278 may be an elastomeric hinge or a plastic living hinge. Another possibility is for the intermediate section 266 to be made of a rigid material.

It is noted that the material of components of the above described attachment mechanisms may be made of any suitable material or materials having the one or more material properties necessary to perform the function of that component. If the functional requirement(s) of the component allow, the component may be made of the same material as the strap portion of the band (or of course made be made of a completely different material). For example, it may be possible to make an attachment mechanism component of silicone having a higher Durometer than the strap made of silicone. In such example, the Shore A Durometer of the silicone strap may be 50 ± 5, with the component of the attachment mechanism having a Shore A Durometer of at least about 10 higher than the strap.

It will become readily apparent to those skilled in the art that the above invention has equally applicability to other types of implantable bands. For example, bands are used for the treatment of fecal incontinence.

One such band is described in U.S. Patent 6,461,292 Bands can also be used to treat urinary incontinence. One such band is described in U.S. Patent Application 2003/0105385. Bands can also be used to treat heartburn and/or acid reflux. One such band is described in U.S. Patent 6,470,892. Bands can also be used to treat impotence. One such band is described in U.S. Patent Application 2003/0114729.

Thus, as used herein and in the claims, an implantable band is a band which may be implanted in a position to occlude flow, such as food or body fluids, through an anatomical passageway, such as a stomach or lumen.

In summary, numerous benefits have been described which result from employing the concepts of the invention. The foregoing description of one or more embodiments of the invention has been presented for purposes of illustration and description. It is not intended to be exhaustive or to limit the invention to the precise form disclosed. Obvious modifications or variations are possible in light of the above teachings. The one or more embodiments were chosen and described in order to best illustrate the principles of the invention and its practical application to thereby enable one of ordinary skill in the art to best utilize the invention in various embodiments and with various modifications as are suited to the particular use contemplated. It is intended that the scope of the invention be defined by the claims appended hereto.

## Claims

1. An implantable band (70, 254, 258) for treatment of a medical condition, the band comprising:
(a) an inextensible strap (16, 32, 100) configured to encircle an anatomical passageway, said strap defining a circumferential direction thereabout, said strap having an inner (34) and outer surface (36) said strap comprising material having at least one first material property; (b) an inflatable balloon (18, 38) extending inwardly from adjacent the inner surface of the strap;
(c) first and second end portions (42, 40; 74, 72; 118, 114; 174, 172; 208, 206; 212, 210; 262, 260) disposed at either end of said strap, said first and second end portions including respective inner and outer surfaces which correspond to said inner and outer surfaces of said strap; and
(d) an integral attachment mechanism (110, 170, 204, 256) configured to attach said first end portion (42, 74, 118, 174, 208, 212, 262) to said second end portion (40, 72, 114, 172 206, 210, 260) so as to secure said strap adjacent the anatomical passageway **characterised in that** said attachment mechanism comprising material having at least one second material property, said at least one second material property being different from said at least one first material property.

2. The band of claim 1, wherein said attachment mechanism comprises a first portion (88) associated with said first end portion (74, 114, 174, 212), and a second portion (78) associated with said second end portion (72, 112, 172, 210), said first portion comprising a cavity (50, 102, 106, 128, 188, 234).

3. The band of claim 2, wherein said first portion (88) comprises a first member extending laterally from said first end portion (74), at least a part of said cavity (50, 102, 106) being defined by said member.

4. The band of claim 2 or 3, wherein said second portion comprises a second member (78) extending laterally from said second end portion (72), said second member defining a passageway configured to have said first end portion (74) and said first member (88) passed therethrough, said first member (88) and said second member (78) configured to cooperate with each other to resist withdrawal of said first member and said first end portion from said passageway.

5. The band of claim 2 or 3, comprising a resilient member (98, 104, 196) at least partially disposed within said cavity (102, 106, 188).

6. The band of claim 5, wherein said resilient member (98, 104, 196) comprises said second material.

7. The band of claim 5, wherein said second portion comprises a second member extending laterally from said second end portion, said second member defining a passageway configured to have said first end portion (108) and said first member passed therethrough, said first member and said second member configured to cooperate with each other to resist withdrawal of said first member and said first end portion from said passageway.

8. The band of claim 5, wherein said cavity (188) is configured to receive said second portion, said second portion comprising at least one groove (178), said resilient member (196) located relative to said cavity to engage said at least one groove (178) when said second portion is disposed at least partially within said cavity (188) at a first position.

9. The band of claim 8, wherein said first portion comprises two spaced apart openings (190, 192) which communicate with said cavity (188), and wherein said resilient member (196) comprises a generally U shaped spring having two spaced apart legs (198, 200) extending from a base (202), said base being disposed within said first portion (174) and each of said spaced apart legs being disposed at least partially within the respective opening.

10. The band of claim 9 wherein said second end comprises at least one inclined surface (176) configured to move said legs apart as said second end is being moved to said first position.

11. The band of claim 2 or 3, wherein said cavity (50) comprises plurality of spaced apart retention members (58).

12. The band of claim 11, wherein said retention members (58) comprise annular rings.

13. The band of claim 11, wherein said second portion (40) comprises a cylindrical portion.

14. The band of 13, wherein said cylindrical portion comprises at least one engagement member (46) configured to cooperate with said cavity (50) to resist axial withdrawal of said second portion (40) from said cavity (50).

15. The band of claim 2 or 3, comprising a plastically deformable member disposed adjacent said cavity.

16. The band of claim 15, wherein said cavity comprises plurality of spaced apart retention members.

17. The band of claim 16, wherein said retention members comprise annular rings.

18. The band of claim 15, wherein said first member comprises two spaced apart legs.

19. The band of claim 2, wherein said first portion comprises a first member extending in a longitudinal direction, said first member comprising at least two spaced apart side walls which define at least a part of said cavity (128), and wherein said second portion comprises at least one resilient member (116) which is configured to be at least partially disposed in said cavity and to engage at least one of said side walls to resist withdrawal of said first member from said cavity.

20. The band of claim 19, wherein said at least one resilient member (116) comprises first and second spaced apart legs (138, 140).

21. The band of claim 20, wherein each of said legs (138, 140) comprises a respective outwardly extending step (150, 152) and wherein said each of said spaced apart side walls includes a respective opening (134, 136) configured to engage one of said steps so as to resist withdrawal of said first member from said cavity (128).

22. The band of claims 19, 20 or 21, wherein said first member and said second member are insert molded into said first end portion (114) and said second end portion (118), respectively.

23. The band of claim 2, wherein said first portion (226) comprises a first member, at least a part of said cavity (234) being defined by said first member, said cavity comprising a generally cylindrical inner surface (234a) defining an axis, a plurality of spaced apart slots (236) and a plurality of ramps (238, 240), and wherein said second portion (214) comprises a cylindrical member which is rotatable relative to said second end portion, said cylindrical member comprising a plurality of outwardly extending legs (224), said cylindrical member being configured to be inserted into and to rotate within said cavity (234).

24. The band of claim 23, wherein said first portion comprises an axially moveable member (228) disposed within said cavity (234), said axially moveable member being resiliently axially biased so as to bias said cylindrical member in a direction out of said cavity.

25. The band of claim 23, wherein said axially moveable member (228) includes a plurality of inclined and declined ramps (246) configured to engage said cylindrical member (214) and to bias said cylindrical member rotationally.

26. The band of claim 23, wherein at least two of said plurality of ramps (238, 240) are disposed circumferentially between at least two adjacent slots of the plurality of said slots (236), said cavity comprising at least one slot disposed between said at least two of said plurality of ramps.

27. The band of any of claims 23 through 26, wherein at least one of said axis and said cylindrical extend in a longitudinal direction.

28. The band of claim 1, wherein said attachment mechanism comprises said first end portion (262) comprising a distal section (268), a proximal section (264) and a textured section (266) intermediate said distal and proximal sections, said distal section being pivotable relative to said textured section (262) and including a distal end (280) comprised of said second material, said distal end (280) comprising at least one laterally extending latch (282), said at least one latch configured to engage said proximal section (264) when said distal section (268) is disposed in a first position overlying said proximal and textured sections with said second end portion (260) disposed inbetween said distal section and said proximal and textured sections.

29. The band of claim 28, wherein said at least one latch (282) is sized to engage -an outer surface of said proximal section (264) such that said distal section urges said second end portion (260) against an inner surface (274) of said textured section.

30. The band of claim 29, wherein the inner surface (274) of said textured section (266) comprises a textured surface configured to engage said second end portion (260) and to resist withdrawal of said second end portion.

31. The band of claim 29, wherein said proximal section (264) includes oppositely facing transverse edges (270), and said at least one latch (282) comprises at least two spaced apart latches, each latch having an inner surfaces (282a), each latch configured to engage one of said transverse edges (270) with its inner surface, each of said transverse edges being configured to resist relative movement of said latches along said transverse edges.

32. The band of claim 31, wherein said transverse edges (270) are textured.

33. The band of claim 32, wherein said transverse edges (270) each comprise a plurality of laterally oriented ridges.

34. The band of claim 29, wherein the inner surface (272) of said proximal section comprises a textured surface configured to engage said second end portion (260) and to resist withdrawal of said second end portion.

35. The band of claim 29, comprising a hinge (278) between said textured section (266) and said distal section (268).

36. The band of claim 35, wherein said hinge (278) is an elastomeric hinge.

37. The band of claim 35, wherein said hinge (278) is a living hinge.

38. The band of claim 35, wherein said hinge is configured such that distal end section (268) adjacent said hinge urges said second end portion (260) against said inner surface (274) of said textured section adjacent said hinge.

39. The band of any of claims 28 to 38, wherein said first end portion (262) comprises a respective release slot (276) associated with a respective one of said at least one latch, each said release slot being configured to allow its associated latch to pass therethrough without significant resistance.

40. The band of claim 39, wherein each said release slot (276) is disposed adjacent said proximal section (264).

41. The band of claim 1, wherein said attachment mechanism comprises a first portion associated with said first end portion, and a second portion associated with said second end portion, said first portion comprising a resilient member.

42. The band of claim 41, wherein said first portion comprises a first member extending laterally from said first end portion, at least a portion of said member comprising said second material.

43. The band of claim 42, wherein said second portion comprises a second member extending laterally from said second end portion, said second member defining a passageway configured to have said first end portion and said first member passed therethrough, said first member and said second member configured to cooperate with each other to resist withdrawal of said first member and said first end portion from said passageway.

## Patentansprüche

1. Implantierbares Band (70, 254, 258) für die Behandlung eines medizinischen Zustands, wobei das Band aufweist:
(a) einen nicht verlängerbaren Streifen (16, 32, 100), der so gestaltet ist, dass er einen anatomischen Durchlass umgibt, wobei der Streifen um diesen eine Umfangsrichtung definiert, wobei der Streifen eine Innen-(34) und eine Außenfläche (36) hat, wobei der Streifen Material aufweist, das wenigstens eine erste Materialeigenschaft hat;
(b) einen aufweitbaren Ballon (18, 38), der sich von angrenzend an die Innenfläche des Streifens nach Innen erstreckt;
(c) einen ersten und einen zweiten Endbereich (42, 40; 74, 72; 118, 114; 174, 172; 208, 206; 212, 210; 262, 260), die an jedem Ende des Streifens angeordnet sind, wobei der erste und der zweite Endbereich jeweilige Innen- und Außenflächen umfassen, die der Innen- und der Außenfläche des Streifens entsprechen; und
(d) einen integralen Befestigungsmechanismus (110, 170, 204, 256), der so gestaltet ist, dass er den ersten Endbereich (42, 74, 118, 174, 208, 212, 262) an dem zweiten Endbereich (40, 72, 114, 172, 206, 210, 260) befestigt, um so den Streifen angrenzend an dem anatomischen Durchlass zu sichern, **dadurch gekennzeichnet, dass** der Befestigungsmechanismus Material mit wenigstens einer zweiten Materialeigenschaft aufweist, wobei die wenigstens eine zweite Materialeigenschaft von der wenigstens einen ersten Materialeigenschaft unterschiedlich ist.

2. Band nach Anspruch 1, bei dem der Befestigungsmechanismus einen ersten Bereich (88), der dem ersten Endbereich (74, 114, 174, 212) zugeordnet ist, und einen zweiten Bereich (78), der dem zweiten Endbereich (72, 112, 172, 210) zugeordnet ist, aufweist, wobei der erste Bereich einen Hohlraum (50, 102, 106, 128, 188, 234) aufweist.

3. Band nach Anspruch 2, bei dem der erste Bereich (88) ein erstes Element aufweist, das sich seitlich von dem ersten Endbereich (74) erstreckt, wobei wenigstens ein Teil des Hohlraums (50, 102, 106) durch das Element definiert ist.

4. Band nach Anspruch 2 oder 3, bei dem der zweite Bereich ein zweites Element (78) aufweist, das sich seitlich von dem zweiten Endbereich (72) erstreckt, wobei das zweite Element einen Durchlass definiert, der so gestaltet ist, dass der erste Endbereich (74) und das erste Element (88) hindurchgeführt werden können, wobei das erste Element (88) und das zweite Element (78) so gestaltet sind, dass sie zusammenwirken, um gegen das Herausziehen des ersten Elements und des ersten Endbereichs aus dem Durchlass Widerstand zu leisten.

5. Band nach Anspruch 2 oder 3, mit einem federnden Element (98, 104, 196), das wenigstens teilweise innerhalb des Hohlraums (102, 106, 188) angeordnet ist.

6. Band nach Anspruch 5, bei dem das federnde Element (98, 104, 196) das zweite Material aufweist.

7. Band nach Anspruch 5, bei dem der zweite Bereich ein zweites Element aufweist, das sich seitlich von dem zweiten Endbereich erstreckt, wobei das zweite Element einen Durchlass definiert, der so gestaltet ist, dass der erste Endbereich (108) und das erste Element hindurchgeführt werden können, wobei das erste Element und das zweite Element so gestaltet sind, dass sie zusammenwirken, um gegen das Herausziehen des ersten Elements und des ersten Endbereichs aus dem Durchlass Widerstand zu leisten.

8. Band nach Anspruch 5, bei dem der Hohlraum (188) so gestaltet ist, dass er den zweiten Bereich aufnimmt, wobei der zweite Bereich wenigstens eine Nut (178) aufweist, wobei das federnde Element (196) in Bezug auf den Hohlraum so angeordnet ist, dass es in die wenigstens eine Nut (178) greift, wenn der zweite Bereich wenigstens teilweise innerhalb des Hohlraums (188) an einer ersten Position angeordnet ist.

9. Band nach Anspruch 8, bei dem der erste Bereich zwei beabstandete Öffnungen (190, 192) aufweist, die mit dem Hohlraum (188) in Verbindung stehen, und bei dem das federnde Element (196) eine im Allgemeinen U-förmige Feder aufweist, die zwei beabstandete Beine (198, 200) hat, die sich von einer Basis (202) erstrecken, wobei die Basis innerhalb des ersten Bereichs (174) angeordnet ist und jedes der beabstandeten Beine wenigstens teilweise innerhalb der jeweiligen Öffnung angeordnet ist.

10. Band nach Anspruch 9, bei dem das zweite Ende wenigstens eine geneigte Fläche (176) aufweist, die so gestaltet ist, dass sie die Beine voneinander weg bewegt, während das zweite Ende in die erste Position bewegt wird.

11. Band nach Anspruch 2 oder 3, bei dem der Hohlraum (50) eine Vielzahl beabstandeter Rückhalteelemente (58) aufweist.

12. Band nach Anspruch 11, bei dem die Rückhalteelemente (58) kreisförmige Ringe aufweisen.

13. Band nach Anspruch 11, bei dem der zweite Bereich (40) einen zylindrischen Bereich aufweist.

14. Band nach Anspruch 13, bei dem der zylindrische Bereich wenigstens ein Eingriffselement (46) aufweist, das so gestaltet ist, dass es mit dem Hohlraum (50) zusammenwirkt, um gegen das axiale Herausziehen des zweiten Bereiches (40) aus dem Hohlraum (50) Widerstand zu leisten.

15. Band nach Anspruch 2 oder 3, mit einem plastisch deformierbaren Element, das angrenzend an den Hohlraum angeordnet ist.

16. Band nach Anspruch 15, bei dem der Hohlraum eine Vielzahl beabstandeter Rückhalteelemente aufweist.

17. Band nach Anspruch 16, bei dem die Rückhalteelemente kreisförmige Ringe aufweisen.

18. Band nach Anspruch 15, bei dem das erste Element zwei beabstandete Beine aufweist.

19. Band nach Anspruch 2, bei dem der erste Bereich ein erstes Element aufweist, das sich in einer Längsrichtung erstreckt, wobei das erste Element wenigstens zwei beabstandete Seitenwände aufweist, die wenigstens einen Teil des Hohlraums (128) definieren, und bei dem der zweite Bereich wenigstens ein federndes Element (116) aufweist, das so gestaltet ist, dass es wenigstens teilweise in dem Hohlraum angeordnet ist und an wenigstens einer der Seitenwände angreift, um gegen das Herausziehen des ersten Elementes aus dem Hohlraum Widerstand zu leisten.

20. Band nach Anspruch 19, bei dem das wenigstens eine federnde Element (116) ein erstes und ein zweites beabstandetes Bein (138, 140) aufweist.

21. Band nach Anspruch 20, bei dem jedes der Beine (138, 140) eine jeweilige sich nach außen erstreckende Stufe (150, 152) aufweist und bei dem jede der beabstandeten Seitenwände eine jeweilige Öffnung (134, 136) umfasst, die so gestaltet ist, dass sie mit einer der Stufen im Eingriff ist, um gegen das Herausziehen des ersten Elementes aus dem Hohlraum (128) Widerstand zu leisten.

22. Band nach Anspruch 19, 20 oder 21, bei dem das erste Element und das zweite Element in den ersten Endbereich (114) bzw. den zweiten Endbereich (118) eingeformt sind.

23. Band nach Anspruch 2, bei dem der erste Bereich (226) ein erstes Element aufweist, wobei wenigstens ein Teil des Hohlraums (234) durch das erste Element definiert ist, wobei der Hohlraum eine im Allgemeinen zylindrische Innenfläche (234a), die eine Achse definiert, eine Vielzahl beabstandeter Schlitze (236) und eine Vielzahl Rampen (238, 240) aufweist, und bei dem der zweite Bereich (214) ein zylindrisches Element aufweist, das in Bezug auf den zweiten Endbereich drehbar ist, wobei das zylindrische Element eine Vielzahl sich nach außen erstreckender Beine (224) aufweist, wobei das zylindrische Element so gestaltet ist, dass es in den Hohlraum (234) eingesetzt werden und sich in ihm drehen kann.

24. Band nach Anspruch 23, bei dem der erste Bereich ein axial bewegbares Element (228) aufweist, das innerhalb des Hohlraums (234) angeordnet ist, wobei das axial bewegbare Element federnd axial vorbelastet ist, um so das zylindrische Element in eine Richtung aus dem Hohlraum hinaus vorzubelasten.

25. Band nach Anspruch 23, bei dem das axial bewegbare Element (228) eine Vielzahl aufsteigender und absteigender Rampen (246) umfasst, die so gestaltet sind, dass sie an dem zylindrischen Element (214) angreifen und das zylindrische Element zur Drehung vorbelasten.

26. Band nach Anspruch 23, bei dem wenigstens zwei aus der Vielzahl der Rampen (238, 240) am Umfang zwischen wenigstens zwei benachbarten Schlitzen aus der Vielzahl der Schlitze (236) angeordnet sind, wobei der Hohlraum wenigstens einen Schlitz aufweist, der zwischen den wenigstens zwei aus der Vielzahl der Rampen angeordnet ist.

27. Band nach einem der Ansprüche 23 bis 26, bei dem wenigstens eines, die Achse oder das zylindrische Element, sich in eine Längsrichtung erstreckt.

28. Band nach Anspruch 1, bei dem der Befestigungsmechanismus den ersten Endbereich (262) mit einem distalen Abschnitt (268), einem proximalen Abschnitt (264) und einem strukturierten Abschnitt (266) zwischen dem distalen und dem proximalen Abschnitt aufweist, wobei der distale Abschnitt in Bezug auf den strukturierten Abschnitt (262) schwenkbar ist und ein distales Ende (280) umfasst, das auf dem zweiten Material besteht, wobei das distale Ende (280) wenigstens eine sich seitlich erstreckende Klinke (282) aufweist, wobei die wenigstens eine Klinke so gestaltet ist, dass sie an dem proximalen Abschnitt (264) angreift, wenn der distale Abschnitt (268) in einer ersten Position angeordnet ist, in der er über dem proximalen und dem strukturierten Abschnitt liegt, wobei der zweite Endbereich (260) zwischen dem distalen Abschnitt und dem proximalen und dem strukturierten Abschnitt liegend angeordnet ist.

29. Band nach Anspruch 28, bei dem die wenigstens eine Klinke (282) so bemessen ist, dass sie an einer Außenfläche des proximalen Abschnitts (264) derart angreift, dass der distale Abschnitt den zweiten Endbereich (260) gegen eine Innenfläche (274) des strukturierten Abschnittes zwingt.

30. Band nach Anspruch 29, bei dem die Innenfläche (274) des strukturierten Abschnitts (266) eine strukturierte Fläche aufweist, die so gestaltet ist, dass sie an dem zweiten Endbereich (260) angreift und gegen das Herausziehen des zweiten Endbereiches Widerstand leistet.

31. Band nach Anspruch 29, bei dem der proximale Abschnitt (264) gegeneinander weisende quer verlaufende Kanten (270) umfasst und die wenigstens eine Klinke (282) wenigstens zwei beabstandete Klinken aufweist, wobei jede Klinke eine Innenfläche (282a) hat, wobei jede Klinke so gestaltet ist, dass sie mit ihrer Innenfläche an einer der quer verlaufenden Kanten (270) angreift, wobei jede der quer verlaufenden Kanten so gestaltet ist, dass sie gegen die relative Bewegung der Klinken entlang den quer verlaufenden Kanten Widerstand leistet.

32. Band nach Anspruch 31, bei dem die quer verlaufenden Kanten (270) strukturiert sind.

33. Band nach Anspruch 32, bei dem die quer verlaufenden Kanten (270) jede eine Vielzahl seitlich ausgerichteter Rippen aufweist.

34. Band nach Anspruch 29, bei dem die Innenfläche (272) des proximalen Abschnitts eine strukturierte Fläche aufweist, die so gestaltet ist, dass sie an dem zweiten Endbereich (260) angreift und gegen das Herausziehen des zweiten Endbereiches Widerstand leistet.

35. Band nach Anspruch 29, das ein Gelenk (278) zwischen dem strukturierten Abschnitt (266) und dem distalen Abschnitt (268) aufweist.

36. Band nach Anspruch 35, bei dem das Gelenk (278) ein elastomeres Gelenk ist.

37. Band nach Anspruch 35, bei dem das Gelenk (278) ein Filmscharnier ist.

38. Band nach Anspruch 35, bei dem das Gelenk so gestaltet ist, dass der distale Endabschnitt (268) angrenzend an das Gelenk den zweiten Endbereich (268) gegen die Innenfläche (274) des strukturierten Abschnitts angrenzend an das Gelenk zwingt.

39. Band nach einem der Ansprüche 28 bis 38, bei dem der erste Endbereich (262) einen jeweiligen Freigabeschlitz (276) aufweist, der einer jeweiligen aus der wenigstens einen Klinke zugeordnet ist, wobei jeder Freigabeschlitz so gestaltet ist, dass seiner zugeordneten Klinke möglich ist, ihn ohne wesentlichen Widerstand zu durchqueren.

40. Band nach Anspruch 39, bei dem jeder Freigabeschlitz (276) angrenzend an den proximalen Abschnitt (264) angeordnet ist.

41. Band nach Anspruch 1, bei dem der Befestigungsmechanismus einen ersten Bereich, der dem ersten Endbereich zugeordnet ist, und einen zweiten Bereich, der dem zweiten Endbereich zugeordnet ist, aufweist, wobei der erste Bereich ein federndes Element aufweist.

42. Band nach Anspruch 41, bei dem der erste Bereich ein erstes Element, das sich seitlich von dem ersten Endabschnitt erstreckt, aufweist, wobei wenigstens ein Teil des Elementes das zweite Material aufweist.

43. Band nach Anspruch 42, bei dem der zweite Bereich eines zweites Element aufweist, das sich seitlich von dem zweiten Endbereich erstreckt, wobei das zweite Element einen Durchlass definiert, der so gestaltet ist, dass durch ihn der erste Endbereich und das erste Element verlaufen können, wobei das erste Element und das zweite Element so gestaltet sind, dass sie zusammenwirken, um gegen das Herausziehen des ersten Elementes und des ersten Endbereiches aus dem Durchlass Widerstand zu leisten.

## Revendications

1. Bande implantable (70, 254, 258) pour le traitement d'un état médical, la bande comprenant :
(a) une sangle non extensible (16, 32, 100) configurée pour encercler une voie de passage anatomique, ladite sangle définissant une direction circonférentielle autour de celle-ci, ladite sangle ayant une surface interne (34) et une surface externe (36), ladite sangle comprenant un matériau ayant au moins une première propriété de matériau ;
(b) un ballonnet gonflable (18, 38) s'étendant vers l'intérieur à partir de la partie adjacente de la surface interne de la sangle ;
(c) des première et seconde parties d'extrémité (42, 40 ; 74, 72 ; 118, 114 ; 174, 172 ; 208, 206 ; 212, 210 ; 262, 260) disposées à chaque extrémité de ladite sangle, lesdites première et seconde parties d'extrémité comprenant des surfaces interne et externe respectives qui correspondent auxdites surfaces interne et externe de ladite sangle ; et
(d) un mécanisme de fixation solidaire (110, 170, 204, 256) configuré pour fixer ladite première partie d'extrémité (42, 74, 118, 208, 212, 262) sur ladite seconde partie d'extrémité (40, 72, 114, 172, 206, 210, 260) afin de fixer ladite sangle de manière adjacente à la voie de passage anatomique, **caractérisée en ce que** ledit mécanisme de fixation comprenant un matériau qui a au moins une seconde propriété de matériau, ladite au moins une seconde propriété de matériau étant différente de ladite au moins une première propriété de matériau.

2. Bande selon la revendication 1, dans laquelle ledit mécanisme de fixation comprend une première partie (88) associée à ladite première partie d'extrémité (74, 114, 174, 212) et une seconde partie (78) associée à ladite seconde partie d'extrémité (72, 112, 172, 210), ladite première partie comprenant une cavité (50, 102, 106, 128, 188, 234).

3. Bande selon la revendication 2, dans laquelle ladite première partie (88) comprend un premier élément s'étendant latéralement à partir de ladite première partie d'extrémité (74), au moins une partie de ladite cavité (50, 102, 106) étant définie par ledit élément.

4. Bande selon la revendication 2 ou 3, dans laquelle ladite seconde partie comprend un second élément (78) s'étendant latéralement à partir de ladite seconde partie d'extrémité (72), ledit second élément définissant une voie de passage configurée pour avoir ladite première partie d'extrémité (74) et ledit premier élément (88) qui passent à travers celle-ci, ledit premier élément (88) et ledit second élément (78) étant configurés pour coopérer l'un avec l'autre pour résister au retrait dudit premier élément et de ladite première partie d'extrémité de ladite voie de passage.

5. Bande selon la revendication 2 ou 3, comprenant un élément résistant (98, 104, 196) au moins partiellement disposé à l'intérieur de ladite cavité (102, 106, 188).

6. Bande selon la revendication 5, dans laquelle ledit élément résistant (98, 104, 196) comprend ledit second matériau.

7. Bande selon la revendication 5, dans laquelle ladite seconde partie comprend un second élément s'étendant latéralement à partir de ladite seconde partie d'extrémité, ledit second élément définissant une voie de passage configurée pour avoir la première partie d'extrémité (108) et ledit premier élément qui passent à travers celle-ci, ledit premier élément et ledit second élément étant configurés pour coopérer l'un avec l'autre afin de résister au retrait dudit premier élément et de ladite première partie d'extrémité de ladite voie de passage.

8. Bande selon la revendication 5, dans laquelle ladite cavité (188) est configurée pour recevoir ladite seconde partie, ladite seconde partie comprenant au moins une rainure (178), ledit élément résistant (196) étant positionné par rapport à ladite cavité pour mettre en prise ladite au moins une rainure (178) lorsque ladite seconde partie est disposée au moins partiellement à l'intérieur de ladite cavité (188) dans une première position.

9. Bande selon la revendication 8, dans laquelle ladite première partie comprend deux ouvertures (190, 192) espacées qui communiquent avec ladite cavité (188), et dans laquelle ledit élément résistant (196) comprend un ressort généralement en forme de U ayant deux pattes (198, 200) espacées s'étendant à partir d'une base (202), ladite base étant disposée à l'intérieur de ladite première partie (174) et chacune desdites pattes espacées étant disposée au moins partiellement à l'intérieur de l'ouverture respective.

10. Bande selon la revendication 9, dans laquelle ladite seconde extrémité comprend au moins une surface inclinée (176) configurée pour écarter lesdites pattes lorsque ladite seconde extrémité est déplacée dans ladite première position.

11. Bande selon la revendication 2 ou 3, dans laquelle ladite cavité (50) comprend une pluralité d'éléments de retenue (58) espacés.

12. Bande selon la revendication 11, dans laquelle lesdits éléments de retenue (58) comprennent des bagues annulaires.

13. Bande selon la revendication 11, dans laquelle ladite seconde partie (40) comprend une partie cylindrique.

14. Bande selon la revendication 13, dans laquelle ladite partie cylindrique comprend au moins un élément de mise en prise (46) configuré pour coopérer avec ladite cavité (50) afin de résister au retrait axial de ladite seconde partie (40) de ladite cavité (50).

15. Bande selon la revendication 2 ou 3, comprenant un élément plastiquement déformable disposé de manière adjacente à ladite cavité.

16. Bande selon la revendication 15, dans laquelle ladite cavité comprend une pluralité d'éléments de retenue espacés.

17. Bande selon la revendication 16, dans laquelle lesdits éléments de retenue comprennent des bagues annulaires.

18. Bande selon la revendication 15, dans laquelle le premier élément comprend deux pattes espacées.

19. Bande selon la revendication 2, dans laquelle ladite première partie comprend un premier élément s'étendant dans une direction longitudinale, ledit premier élément comprenant au moins deux parois latérales espacées qui définissent au moins une partie de ladite cavité (128), et dans laquelle ladite seconde partie comprend au moins un élément résistant (116) qui est configuré pour être au moins partiellement disposé dans ladite cavité et pour mettre en prise au moins l'une desdites parois latérales pour résister au retrait dudit premier élément de ladite cavité.

20. Bande selon la revendication 19, dans laquelle ledit au moins un élément résistant (116) comprend les première et seconde pattes (138, 140) espacées.

21. Bande selon la revendication 20, dans laquelle chacune desdites pattes (138, 140) comprend un échelon (150,152) respectif s'étendant vers l'extérieur, et dans laquelle chacune desdites parois latérales espacées comprend une ouverture (134, 136) respective configurée pour mettre en prise l'un desdits échelons afin de résister au retrait dudit premier élément de ladite cavité (128).

22. Bande selon les revendications 19, 20 ou 21, dans laquelle ledit premier élément et ledit second élément sont moulés par insertion dans ladite première partie d'extrémité (114) et ladite seconde partie d'extrémité (118), respectivement.

23. Bande selon la revendication 2, dans laquelle ladite première position (226) comprend un premier élément, au moins une partie de ladite cavité (234) étant définie par ledit premier élément, ladite cavité comprenant une surface interne (234a) généralement cylindrique définissant un axe, une pluralité de fentes (236) espacées et une pluralité de rampes (238, 240), et dans laquelle ladite seconde partie (214) comprend un élément cylindrique qui peut tourner par rapport à ladite seconde partie d'extrémité, ledit élément cylindrique comprenant une pluralité de pattes (224) s'étendant vers l'extérieur, ledit élément cylindrique étant configuré pour être inséré dans et pour tourner à l'intérieur de ladite cavité (234).

24. Bande selon la revendication 23, dans laquelle ladite première partie comprend un élément (228) axialement mobile disposé à l'intérieur de ladite cavité (234), ledit élément axialement mobile étant sollicité axialement de manière résistant afin de solliciter ledit élément cylindrique dans une direction hors de ladite cavité.

25. Bande selon la revendication 23, dans laquelle ledit élément axialement mobile (228) comprend une pluralité de rampes inclinées et en pente (246) configurées pour mettre en prise ledit élément cylindrique (214) et pour solliciter ledit élément cylindrique en rotation.

26. Bande selon la revendication 23, dans laquelle au moins deux de ladite pluralité de rampes (238, 240) sont disposées de manière circonférentielle entre au moins deux fentes adjacentes de la pluralité desdites fentes (236), ladite cavité comprenant au moins une fente disposée entre lesdites au moins deux rampes de ladite pluralité de rampes.

27. Bande selon l'une quelconque des revendications 23 à 26, dans laquelle au moins l'un parmi ledit axe et ledit cylindre s'étend dans une direction longitudinale.

28. Bande selon la revendication 1, dans laquelle ledit mécanisme de fixation comprend ladite première partie d'extrémité (262) comprenant une section distale (268), une section proximale (264) et une section texturée (266) entre lesdites sections distale et proximale, ladite section distale pouvant pivoter par rapport à ladite section texturée (262) et comprenant une extrémité distale (280) composée dudit second matériau, ladite extrémité distale (280) comprenant au moins un verrou (282) s'étendant latéralement, ledit au moins un verrou étant configuré pour mettre en prise ladite section proximale (264) lorsque ladite section distale (268) est disposée dans une première position recouvrant lesdites sections proximale et texturée avec ladite seconde partie d'extrémité (260) disposée entre ladite section distale et lesdites sections proximale et texturée.

29. Bande selon la revendication 28, dans laquelle ledit au moins un verrou (282) est dimensionné pour mettre en prise une surface externe de ladite section proximale (264) de sorte que ladite section distale pousse ladite seconde partie d'extrémité (260) contre une surface interne (274) de ladite section texturée.

30. Bande selon la revendication 29, dans laquelle la surface interne (274) de ladite section texturée (266) comprend une surface texturée configurée pour mettre en prise ladite seconde partie d'extrémité (260) et pour résister au retrait de ladite seconde partie d'extrémité.

31. Bande selon la revendication 29, dans laquelle ladite section proximale (264) comprend des bords transversaux (270) se faisant face de manière opposée, et ledit au moins un verrou (282) comprend au moins deux verrous espacés, chaque verrou ayant une surface interne (282a), chaque verrou étant configuré pour mettre en prise l'un desdits bords transversaux (270) avec sa surface interne, chacun desdits bords transversaux étant configuré pour résister au mouvement relatif desdits verrous le long desdits bords transversaux.

32. Bande selon la revendication 31, dans laquelle lesdits bords transversaux (270) sont texturés.

33. Bande selon la revendication 32, dans laquelle lesdits bords transversaux (270) comprennent chacun une pluralité de parties saillantes orientées latéralement.

34. Bande selon la revendication 29, dans laquelle la surface interne (272) de ladite section proximale comprend une surface texturée configurée pour mettre en prise ladite seconde partie d'extrémité (260) et pour résister au retrait de ladite seconde partie d'extrémité.

35. Bande selon la revendication 29, comprenant une charnière (278) entre ladite section texturée (266) et ladite section distale (268).

36. Bande selon la revendication 35, dans laquelle ladite charnière (278) est une charnière en élastomère.

37. Bande selon la revendication 35, dans laquelle ladite charnière (278) est une charnière active.

38. Bande selon la revendication 35, dans laquelle ladite charnière est configurée de sorte que la section d'extrémité distale (268) adjacente à ladite charnière pousse ladite seconde partie d'extrémité (260) contre ladite surface interne (274) de ladite section texturée adjacente à ladite charnière.

39. Bande selon l'une quelconque des revendications 28 à 38, dans laquelle ladite première partie d'extrémité (262) comprend une fente de déblocage (276) respective associée à un verrou respectif dudit au moins un verrou, chacune desdites fentes de déblocage étant configurée pour permettre à son verrou associé de passer à travers celle-ci sans résistance significative.

40. Bande selon la revendication 39, dans laquelle chacune desdites fentes de déblocage (276) est disposée de manière adjacente à ladite section proximale (264).

41. Bande selon la revendication 1, dans laquelle ledit mécanisme de fixation comprend une première partie associée à ladite première partie d'extrémité et une seconde partie associée à ladite seconde partie d'extrémité, ladite première partie comprenant un élément résistant.

42. Bande selon la revendication 41, dans laquelle ladite première partie comprend un premier élément s'étendant latéralement à partir de ladite première partie d'extrémité, au moins une partie dudit élément comprenant ledit second matériau.

43. Bande selon la revendication 42, dans laquelle ladite seconde partie comprend un second élément s'étendant latéralement à partir de ladite seconde partie d'extrémité, ledit second élément définissant une voie de passage configurée pour avoir ladite première partie d'extrémité et ledit premier élément qui passent à travers celle-ci, ledit premier élément et ledit second élément étant configurés pour coopérer l'un avec l'autre pour résister au retrait dudit premier élément et de ladite première partie d'extrémité de ladite voie de passage.
